(19) Europäisches Patentamt
European Patent Office
Office européen des brevets

(11) Veröffentlichungsnummer: **0 123 112**
**B1**

(12) # EUROPÄISCHE PATENTSCHRIFT

(45) Veröffentlichungstag der Patentschrift: **21.09.88**

(51) Int. Cl.⁴: **C 07 D 405/04**, C 07 D 409/04, C 07 D 405/14, A 61 K 31/445

(21) Anmeldenummer: **84102903.6**

(22) Anmeldetag: **16.03.84**

(54) **Chromon- und thiochromonsubstituierte 1,4-Dihydropyridinderivate, mehrere Verfahren zu ihrer Herstellung sowie ihre Verwendung in Arzneimitteln.**

(30) Priorität: **25.03.83 DE 3311005**

(43) Veröffentlichungstag der Anmeldung: **31.10.84 Patentblatt 84/44**

(45) Bekanntmachung des Hinweises auf die Patenterteilung: **21.09.88 Patentblatt 88/38**

(84) Benannte Vertragsstaaten: **AT BE CH DE FR GB IT LI LU NL SE**

(56) Entgegenhaltungen:
**EP-A-0 157 324**
**GB-A-1 042 480**

(73) Patentinhaber: **BAYER AG, Konzernverwaltung RP Patentabteilung, D-5090 Leverkusen 1 Bayerwerk (DE)**

(72) Erfinder: **Goldmann, Siegfried, Dr., Kuckuckstrasse 41, D-5600 Wuppertal 2 (DE)**
Erfinder: **Franckowiak, Gerhard, Dr., Henselweg 10, D-5600 Wuppertal 1 (DE)**
Erfinder: **Schramm, Matthias, Dr., Paffrather Strasse 38, D-5000 Köln (DE)**
Erfinder: **Thomas, Günter, Dr., Claudiusweg 9, D-5600 Wuppertal 1 (DE)**
Erfinder: **Gross, Rainer, Dr., Platzhoffstrasse 23, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1988

**Beschreibung**

Die vorliegende Erfindung betrifft neue 1,4-Dihydropyridine, mehrere Verfahren zu ihrer Herstellung sowie ihre Verwendung in Arzneimitteln, insbesondere in kreislaufbeeinflussenden Arzneimitteln.

Die neuen Dihydropyridine sind durch folgende allgemeine Formel I gekennzeichnet,

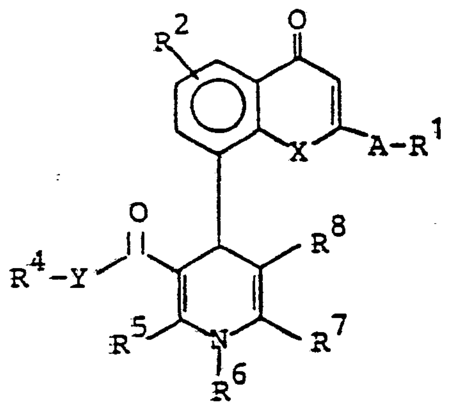

(I)

in welcher

$R^1$ für Wasserstoff, einen geradkettigen, cyclischen oder verzweigten aliphatischen Kohlenwasserstoff mit 1 bis 8 C-Atomen, einen Carbonsäurealkylester mit 1 bis 8 C-Atomen in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei die genannten Aryle oder Heteroaryle gegebenenfalls 1 bis 5 gleiche oder verschiedene Substituenten aus der Gruppe Fluor, Chlor, Brom, Jod, Alkyl (1 bis 8 C-Atome), Alkylthio (1 bis 8 C-Atome), Alkylsulfinyl (1 bis 8 C-Atome), Cyano, Hydroxy, Nitro, Mono- oder Polyfluoralkyl (1 bis 4 C-Atome), Mono- oder Polyfluoralkoxy (1 bis 4 C-Atome), Mono- oder Polyfluoralkylthio (1 bis 4 C-Atome), Amino, Monoalkylamino (1 bis 5 C-Atome), Dialkylamino (jeweils 1 bis 5 C-Atome) aufweisen,

$R^2$ für Wasserstoff oder ein bis drei Fluor- oder Chloratome steht,

$R^4$ für einen geradkettigen, verzweigten oder cyclischen gesättigten oder ungesättigten Kohlenwasserstoffrest mit 1 bis 18 C-Atomen steht, der gegebenenfalls substituiert ist durch Alkoxy (mit 1 bis 8 C-Atomen), Alkylthio (mit 1 bis 8 C-Atomen), Alkylsulfinyl (mit 1 bis 8 C-Atomen), Trialkylsilyl (mit jeweils 1 bis 5 C-Atomen), Cl, Br, J, F, Cyano, Hydroxy, Amino, Alkylamino (mit 1 bis 5 C-Atomenl, Dialkylamino (mit jeweils 1 bis 5 C-Atomen), Morpholinyl, Piperidyl, Piperazinyl, Nitro, Nitrat, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl oder Chinazolyl, wobei die genannten Aromaten oder Heteroaromaten gegebenenfalls substituiert sein können durch 1 bis 3 gleiche oder verschiedene Substituenten aus der Gruppe F, Cl, Br, Alkyl (mit 1 bis 5 C-Atomen), Alkoxy (mit 1 bis 5 C-Atomen), Alkylthio (mit 1 bis 4 C-Atomen), Alkylsulfinyl (mit 1 bis 4 C-Atomen),

$R^5$ und $R^7$ gleich oder verschieden sein können und für Wasserstoff oder einen geradkettigen, verzweigten oder cyclischen, gesättigten oder ungesättigten aliphatischen Alkylrest (1 bis 8 C-Atome) stehen, der gegebenenfalls ein oder zwei gleiche oder verschiedene Heterokettenglieder aus der Gruppe O, CO, S oder N-Alkyl (1 bis 6 C-Atome) enthält und der gegebenenfalls substituiert ist durch Halogen, Nitro, Cyano, Hydroxy, Amino, Monoalkylamino oder Dialkylamino (mit jeweils 1 bis 5 C-Atomen),

$R^6$ für Wasserstoff oder einen geradkettigen oder verzweigten Alkylrest (mit 1 bis 16 C-Atomen) steht, der gegebenenfalls substituiert ist durch Alkoxy (mit 1 bis 8 C-Atomen), Halogen oder Morpholino,

$R^8$ für Wasserstoff, Nitro, Cyano, Fluor, Chlor, Brom, Alkyl (1 bis 3 C-Atome), Mono- oder Polyfluoralkyl (1 bis 3 C-Atome) oder Hydroxycarbonyl steht,

A für eine einfache Bindung oder eine Alkylenkette (1 bis 18 C-Atome) steht, die gegebenenfalls durch O oder S unterbrochen sein kann,

X für O oder S steht und

Y für eine einfache Bindung, O, S, -NH- oder -N(Alkyl)- (mit 1 bis 6 C-Atomen) steht.

in Form von Isomeren, Isomerengemischen, Racematen und optischen Antipoden sowie ihre pharmazeutisch unbedenklichen Salze.

Als Salze seien beispielsweise genannt Hydrochloride, Hydrogensulfate, Sulfate, Hydrogenphosphate, Acetate, Maleate, Benzoate, Citronate, Tartrate oder Lactate.

Vorzugsweise seien solche Verbindungen der allgemeinen Formel (I) genannt, in welcher

$R^1$ für Wasserstoff, einen geradkettigen, verzweigten oder cyclischen aliphatischen Kohlenwasserstoffrest mit 1 bis 7 C-Atomen, einen Carbonsäurealkylester mit 1 bis 6 C-Atomen im Alkylrest, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl, Benzimidazolyl oder Chinazolyl steht, wobei die genannten Aryle bzw. Heteroaryle gegebenenfalls 1 bis 4 gleiche oder verschiedene Substituenten aus der Gruppe Fluor, Chlor, Brom, Alkyl (1 bis 6 C-Atome), Alkylthio (1 bis 6 C-Atome), Alkylsulfinyl (1 bis 6 C-Atome), Cyano, Hydroxy, Nitro, Mono- oder

Polyfluoralkyl (1 bis 3 C-Atome), Mono- oder Polyfluoralkoxy (1 bis 3 C-Atome), Amino, Monoalkylamino (1 bis 4 C-Atome), Dialkylamino (jeweils 1 bis 4 C-Atome) aufweisen,

$R^2$ für Wasserstoff oder ein bis drei Fluoratome steht,

$R^4$ für einen geradkettigen, verzweigten oder cyclischen, gesättigten oder ungesättigten Kohlenwasserstoffrest mit 1 bis 12 C-Atomen steht, der gegebenenfalls substituiert ist durch Alkoxy (mit 1 bis 6 C-Atomen), Alkylthio (mit 1 bis 6 C-Atomen), Alkylsulfinyl (mit 1 bis 6 C-Atomen), Trialkylsilyl (mit jeweils 1 bis 3 C-Atomen), Cl, Br, F, Cyano, Hydroxy, Amino, Alkylamino (mit 1 bis 4 C-Atomen), Dialkylamino (mit jeweils 1 bis 4 C-Atomen),

Morpholinyl, Piperidyl, Piperazinyl, Nitro, Nitrat, Phenyl, Naphthyl, Thienyl, Furyl, Pyrryl, Imidazolyl, Oxazolyl, Thiazolyl, Pyridyl, Pyrimidyl, Pyrazinyl, Indolyl, wobei die genannten Aromaten oder Heteroaromaten gegebenenfalls substituiert sein können durch 1 bis 3 gleiche oder verschiedene Substituenten aus der Gruppe F, Cl, Br, Alkyl (mit 1 bis 4 C-Atomen), Alkoxy (mit 1 bis 4 C-Atomen), Alkylthio (mit 1 bis 3 C-Atomen), Alkylsulfinyl (mit 1 bis 3 C-Atomen),

$R^5$ und $R^7$ gleich oder verschieden sind und für Wasserstoff oder einen geradkettigen, verzweigten oder cyclischen, gesättigten oder ungesättigten aliphatischen Alkylrest (1 bis 6 C-Atome) stehen, der gegebenenfalls ein oder zwei gleiche oder verschiedene Heterokettenglieder aus der Gruppe O, CO, S oder N-Alkyl (1 bis 4 C-Atome) enthält und der gegebenenfalls substituiert ist durch Halogen, Nitro, Cyano, Hydroxy, Amino, Monoalkylamino (mit 1 bis 4 C-Atome) oder Dialkylamino (mit jeweils 1 bis 4 C-Atomen),

$R^6$ für Wasserstoff oder einen geradkettigen oder verzweigten Alkylrest (mit 1 bis 12 C-Atomen) steht, der gegebenenfalls substituiert ist durch Alkoxy (mit 1 bis 6 C-Atomen), Halogen oder Morpholino,

$R^8$ für Nitro, Cyano, Fluor oder Chlor steht,

A für eine einfache Bindung oder eine Alkylenkette (1 bis 16 C-Atome) steht, die gegebenenfalls durch O oder S unterbrochen sein kann,

X für O oder S steht und

Y für eine einfache Bindung, O, S, -NH- oder -N(Alkyl)- (mit 1 bis 4 C-Atomen) steht.

Die erfindungsgemäßen Verbindungen der allgemeinen Formel (I) können hergestellt werden indem man

A) Aldehyde der allgemeinen Formel (II)

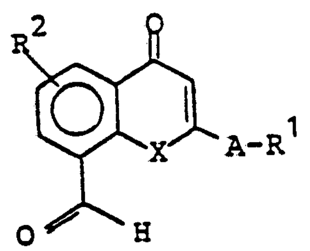

(II)

in welcher $R^1$, $R^2$, A und X die oben angegebene Bedeutung haben mit Enaminen der allgemeinen Formel (III)

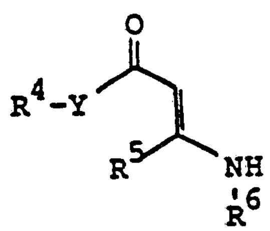

(III)

in welcher $R^4$, $R^5$, $R^6$ und Y die oben angegebene Bedeutung haben und Ketonen der allgemeinen Formel (IV) in welcher

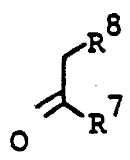

(IV)

$R^7$ und $R^8$ die oben angegebene Bedeutung haben

ggf. in Gegenwart inerter organischer Lösungsmittel bei Temperaturen zwischen 20 und 150° C umsetzt oder

B) Aldehyde der allgemeinen Formel (II) in welcher $R^1$, $R^2$, A und X die oben angegebene Bedeutung haben mit Ketonen der allgemeinen Formel (V)

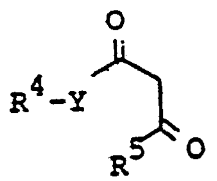

(V)

in welcher $R^4$, $R^5$ und Y die oben angegebene Bedeutung haben und Enaminen der allgemeinen Formel (VI)

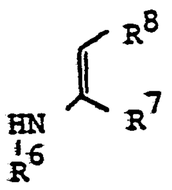

(VI)

in welcher $R^6$, $R^7$ und $R^8$ die oben angegebene Bedeutung haben, gegebenenfalls in Gegenwart inerter organischer Lösungsmittel bei Temperaturen zwischen 20 und 150°C umsetzt, oder

C) Ylidenverbindungen der allgemeinen Formel (VII)

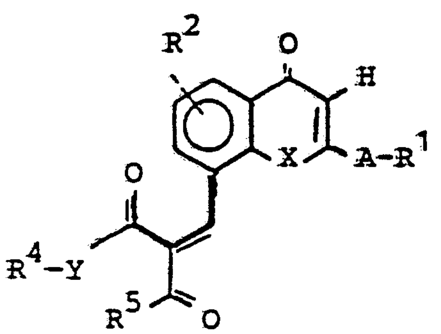

(VII)

in welcher $R^1$, $R^2$, $R^4$, $R^5$ A, X, und Y die oben angegebene Bedeutung haben, mit Enaminen der allgemeinen Formel (VI) in welcher $R^6$, $R^7$ und $R^8$ die oben angegebene Bedeutung haben, gegebenenfalls in Gegenwart inerter organischer Lösungsmittel bei Temperaturen zwischen 20 und 150°C umsetzt

oder

D) Benzylidenverbindungen der allgemeinen Formel (VIII)

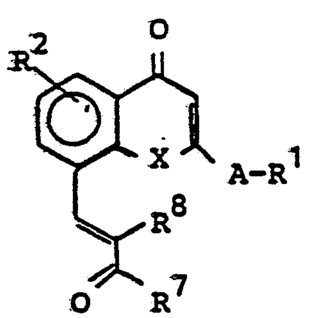

(VIII)

in welcher $R^1$, $R^2$, $R^7$, $R^8$, A und X die oben angegebene Bedeutung haben mit Enaminen der allgemeinen Formel (III) in welcher $R^4$, $R^5$, $R^6$ und Y die oben angegebene Bedeutung haben, gegebenenfalls in inerten organischen Lösungsmitteln bei Temperaturen von 20°C bis 150°C umsetzt.

Die Verfahrensvariante A wird bevorzugt, falls der Rest $R^8$ Nitro darstellt, die Verfahrensvarianten B und C werden bevorzugt, falls der Rest $R^8$ Nitril darstellt, und die Verfahrensvariante D wird bevorzugt falls der Rest $R^8$ die verbleibenden Bedeutungen darstellt.

Das Mengenverhältnis der Reaktanden zueinander ist beliebig, bevorzugt werden jedoch äquimolare Mengen eingesetzt. In der Verfahrensvariante A können auch die Verbindungen der Formeln III oder IV, in der

Verfahrensvariante B die Verbindungen der Formeln V oder VI, in der Verfahrensvariante C die Verbindungen der Formel VI und in der Verfahrensvariante D die Verbindung der Formel III im Überschuß bis zu 3 Molen eingesetzt werden.

Die Umsetzungstemperaturen in allen Verfahrensvarianten betragen bevorzugt 30 bis 120°C, insbesondere die Siedetemperaturen der benutzten Lösungsmittel.

Wenn in Gegenwart von organischen Lösungsmittel gearbeitet wird; kommen alle inerten Lösungsmittel in Betracht, wie beispielsweise Alkohol, Essigsäure, Benzol und/oder Toluol.

Die zur Herstellung verwendeten Aldehyde der Formel II sind neu und können hergestellt werden (siehe EP-A-123 113), in dem man im Falle X = S Thiochromone der Formel

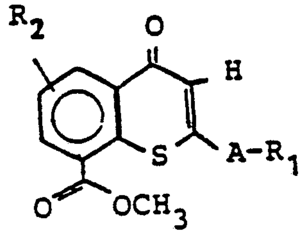

in der

$R_1$, $R_2$ und A die bereits genannten Bedeutungen besitzen, zu Benzylalkoholen reduziert und man den Benzylalkohol

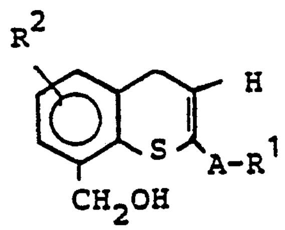

mit Oxidationsmitteln zu Aldehyden oxidiert.

Die als Ausgangsstoffe verwendeten Thiochromone sind bekannt oder können nach bekannten Verfahren hergestellt werden (Bossert, Lieb. Ann. 680, 40 (1964)).

Für die Reduktion zum Benzylalkohol können inerte organische Lösungsmittel eingesetzt werden, beispielsweise Ether wie z. B. Dioxan, Diethylether, Tetrahydrofuran, Dimethoxyethan oder Aromaten wie z. B. Toluol oder Benzol. Als Reduktionsmittel seien beispielhaft Alkalialuminiumhydride wie z. B. $LiALH_4$ oder Alkylaluminiumhydride wie z. B. Diisobutylaluminiumhydrid erwähnt.

Bei der Durchführung dieses Verfahrens arbeitet man vorzugsweise in einem Temperaturbereich von -100°C bis +60°C, insbesondere in einem Bereich von -60°C bis +30°C.

Die Umsetzung erfolgt üblicherweise bei Normaldruck, kann aber auch bei erhöhtem Druck durchgeführt werden.

Das Reduktionsmittel wird in den dem Fachmann geläufigen Mengen zugesetzt, bevorzugt in mindestens vier und höchstens 8 Hydridäquivalenten.

Für die Oxidation des Benzylalkohols zum Aldehyd können die gleichen Lösungsmittel wie bei der Reduktion Verwendung finden, zusätzlich aber auch halogenierte Kohlenwasserstoffe wie Chloroform und Methylenchlorid oder Ketone wie z. B. Aceton.

Als Oxidationsmittel können die üblicherweise für Oxidationen eingesetzten Übergangsmetalloxide eingesetzt werden, vorzugsweise jedoch Mangandioxid.

Bei der Durchführung der Oxidation arbeitet man normalerweise in einem Temperaturbereich von -30 bis +200°C vorzugsweise beim Siedepunkt des jeweiligen Lösungsmittels.

Die Oxidation erfolgt üblicherweise bei Normaldruck, kann aber auch bei erhöhtem Druck durchgeführt werden.

Das Oxidationsmittel kann in 3 bis 20, vorzugsweise in 5 bis 10 Oxidationsäquivalenten eingesetzt werden. Auch kann es zweckmäßig sein, von Zeit zu Zeit frisches Oxidationsmittel zum Reaktionsansatz zuzugeben.

Im Falle X = O werden Chromone der Formel

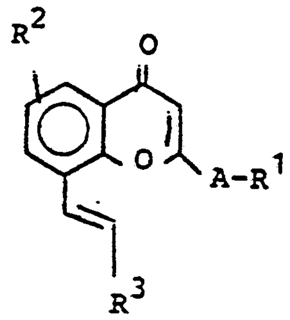

in der

$R^1$, $R^2$ und A die oben angegebene Bedeutung haben, mit der Einschränkung, daß A keine Alkylenkette ist oder Schwefel enthält und

$R^3$ für Wasserstoff oder Alkyl (mit 1 bis 10 C-Atomen) steht, mit Ozon in Anwesenheit inerter organischer Lösungsmittel umsetzt, und anschließend reduktiv aufarbeitet.

Die als Ausgangsstoffe verwendeten 8-Alkenylchromone sind bekannt oder können nach bekannten Verfahren hergestellt werden (US-Patent 3 350 411, vgl. auch Synthesis 1982, 221).

Für die Ozonolyse seien als inerte Lösungsmittel genannt: chlorierte Kohlenwasserstoffe wie z. B. Methylenchlorid, Chloroform oder Tetrachlorkohlenstoff, Ester wie z. B. Essigsäureethylester, Alkohole wie z. B. Methanol oder Ethanol, Säuren wie z. B. Ameisensäure oder Essigsäure.

Die Ozonolyse erfolgt bei -100°C bis 20°C, vorzugsweise jedoch bei -80°C bis -30°C mit anschließender reduktiver Aufarbeitung z. B. mit Dimethylsulfid, Zinkstaub, katalytischer Hydrierung oder Natriumdithionit.

Auf ein Mol Chromon wird nur ein Mol Ozon verwendet um eine Spaltung weiterer Doppelbindungen zu verhindern.

Die zur Herstellung verwendeten Enamine der allgemeinen Formel (III) sind bekannt oder können nach bekannten Verfahren hergestellt werden (vgl. A.C. Cope, J. Am. Chem. Soc. 67, 1017 (1945)).

Die zur Herstellung verwendeten Ketoverbindungen der allgemeinen Formel (IV) sind bekannt oder können nach bekannten Verfahren hergestellt werden (J. Org. Chem. 20, 927 (1955)).

Die zur Herstellung verwendeten Ketone der Struktur (V) sind bekannt oder können nach bekannten Verfahren hergestellt werden: z. B. für Y = O, D. Borrmann, Umsetzung von Diketen mit Alkoholen, Phenolen und Mercaptanen in Houbel-Weyl, Methoden der organischen Chemie, Vol. VII 14, 230 ff. (1968), Y. Dikawa, K. Sugano und O. Yonemitsu, J. Org. Chem. 43, 2087 (1978).

Die zur Herstellung verwendeten Enamine der allgemeinen Formel (VI) sind bekannt oder können nach bekannten Verfahren hergestellt werden.

Die zur Herstellung verwendeten Benzylidenverbindungen der Struktur (VII) sind neu, können aber nach bekannten Verfahren hergestellt werden (vgl. G.Jones "The Knoevenagel Condensation" in Org. Reactions, Vol. XV, 204 ff (1967)).

Die erfindungsgemäßen Verbindungen zeigen ein nicht vorhersehbares und wertvolles pharmakologisches Wirkungsspektrum. Sie können als Cardiotonika zur Verbesserung der Herzkontraktilität dienen. Darüber hinaus können sie dadurch, daß sie den $Ca^{++}$-Einstrom in die Zelle erhöhen, als Antihypotonica, zur Senkung von Blutzucker, zur Abschwellung von Schleimhäuten und zur Beeinflussung des Salz- und Flüssigkeitshaushaltes eingesetzt werden.

Die erfindungsgemäßen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmittel und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermitteln (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder

intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbessern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation, größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Beispiele**

**Beispiel 1**

2,6-Dimethyl-5-nitro-4-(2-phenyl-4-oxo-4H-chromen-8-yl)-1,4-dihydropyridin-3-carbonsäuremethylester

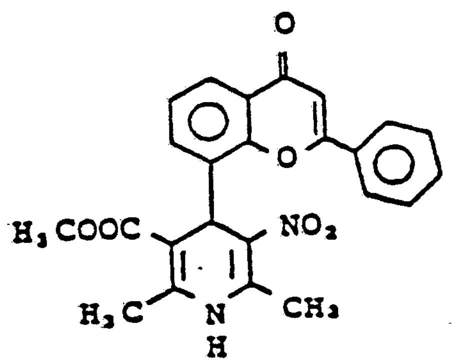

Je 10 mMol 2-Phenyl-4-oxo-4H-chromen-8-aldehyd, 3-Aminocrotonsäuremethylester und Nitroaceton werden in 20 ml abs. EtOH 3 h am Rückfluß gekocht, eingeengt und aus Methanol kristallisiert.

Fp: > 260°C

**Beispiel 2**

5-Cyano-2,6-dimethyl-4-(2-phenyl-4-oxo-4H-thiochromen-8-yl)-1,4-dihydropyridin-3-carbonsäurebutylester

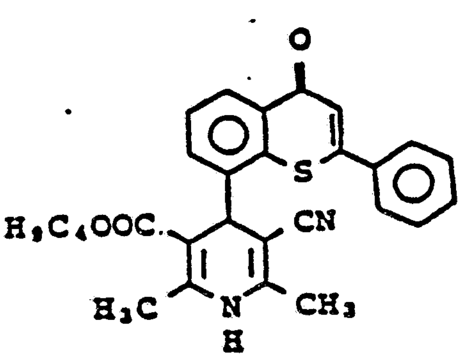

Je 10 mMol 2-Phenyl-4-oxo-4H-thiochromen-8-aldehyd, Acetessigsäurebutylester und 3-Aminocrotonnitril wurden in 30 ml Ethanol über Nacht am Rückfluß gekocht, eingeengt und mit Essigester kristallisiert.
Fp: 197-206°C.

**Beispiel 3**

2,6-Dimethyl-5-nitro-4-(2-phenyl-4-oxo-4H-thiochromen-8-yl)-1,4-dihydropyridin-3-carbonsäuremethylester

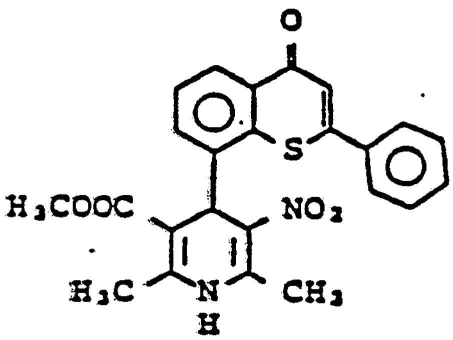

Je 20 mMol 2-Phenyl-4-oxo-4H-thiochromen-8-aldehyd, Nitroaceton und 3-Aminocrotonsäuremethylester wurden in 30 ml Ethanol 3 h am Rückfluß gekocht, eingeengt und mit Toluol/Essigester = 1 : 1 an Kieselgel chromatographiert, wobei der intensiv gelb gefärbte Fleck isoliert wurde.
Fp: 209-211°C (aus Methanol).
Analog Beispiel 1 oder 3 wurden dargestellt:

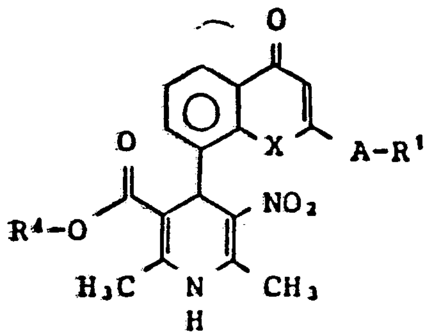

| Beispiel | A-R[1] | R[4] | X | FP (°C) | analog Beispiel |
|---|---|---|---|---|---|
| 4 | 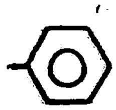 | $-C_2H_5$ | S | 209-11 | 3 |
| 5 | " | $-CH_2-CH_2-CH_3$ | S | 268-70 | 3 |
| 6 | " | $-(CH_2)_3-CH_3$ | S | 209-11 | 3 |
| 7 | " | $-CH_2-CH(CH_3)_2$ | S | 205-07 | 3 |
| 8 | " | $-CH_2-$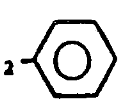 | S | 143-45 | 3 |
| 9 | " | $-(CH_2)_3-CH_3$ | S | >260 | 1 |
| 10 | 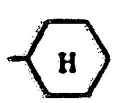 | $-C_2H_5$ | S | >250 | 1 |

| | | | | | |
|---|---|---|---|---|---|
| 11 | 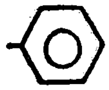 | $-CH_2H_5$ | O | 259 | 1 |
| 12 | " | $C_3H_7(n)$ | O | 251-53 | 1 |
| 13 | " | $C_8H_{17}(n)$ | O | 195-200 | 1 |
| 14 | $-(CH_2)_8-CH_3$ | $C_2H_5$ | O | 151-155 | 1 |
| 15 | $-(CH_2)_8-CH_3$ | $CH_3$ | O | 157-162 | 1 |
| 16 | 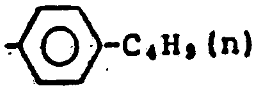 | $-C_2H_5$ | O | 251-152 | 1 |
| 17 | -COOEt | $-CH_3$ | O | 233-236 | 1 |
| 18 | $-C(CH_3)_3$ | $-CH_3$ | O | $>260$ | 1 |
| 19 | $-C(CH_3)_3$ | $-C_2H_5$ | O | 240-244 | 1 |
| 20 | $-C(CH_3)_3$ | $-C_4H_9(n)$ | O | 238-240 | 1 |
| 21 | 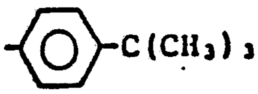 | $-C_2H_5$ | O | 258-260 | 1 |
| 22 | " | $-C_4H_9(n)$ | O | $>270$ | 1 |
| 23 | 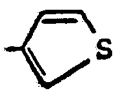 | $C_2H_5$ | O | 247-249 | 1 |
| 24 | " | $C_4H_9(n)$ | O | 245-247 | 1 |
| 25 | $-COOC_2H_5$ | $C_4H_9(n)$ | O | 155 | 1 |
| 26 | $-COOC_2H_5$ | $C_2H_5$ | O | 120-130 | 1 |
| 27 | $-(CH_2)_8-CH_3$ | $C_4H_9(n)$ | O | 155-157 | 1 |
| 28 | $-(CH_2)_8-CH_3$ | $C_8H_{17}(n)$ | O | 128-132 | 1 |
| 29 | $-CH_3$ | $C_2H_5$ | O | 247-248 | 1 |
| 30 | $-CH_3$ | $-CH_3$ | O | $>260$ | 1 |
| 31 | 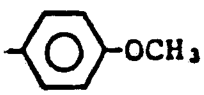 | $-C_4H_9(n)$ | O | $>260$ | 1 |
| 32 | 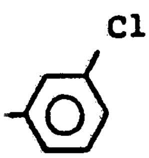 | $-C_4H_9(n)$ | O | 247-249 | 1 |
| 33 | 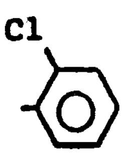 | $-C_2H_5$ | O | 256-257 | 1 |
| 34 | 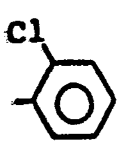 | $-CH_3$ | O | 238-242 | 1 |
| 35 | 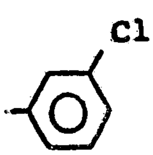 | $-C_2H_5$ | O | 254-257 | 1 |

Analog Beispiel 2 wurden dargestellt:

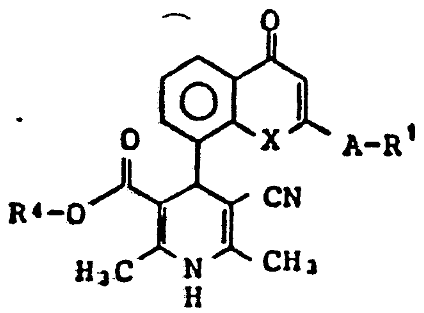

| Beispiel | A-$R^1$ | $R^4$ | X | FP (°C) |
|---|---|---|---|---|
| 36 | 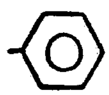 | $-CH_3$ | S | >270 |
| 37 | " | $-CH_2-CH_3$ | S | 260-01 |
| 38 | " | $-(CH_2)_2-CH_3$ | S | 212-15 |
| 39 | " | $-CH_2-CH=CH_2$ | S | 226-28 |
| 40 | " | $-CH_3$ | O | >280 |
| 41 | " | $-C_2H_5$ | O | 267-70 |
| 42 | $(CH_2)_8-CH_3$ | $-C_2H_5$ | O | 174-76 |

**Prüfung der positiv inotropen Wirkung**

Versuchsanordnung

Die linken Vorhöfe von Meerschweinchenherzen werden isoliert und in ein thermostatisiertes Organbad gehängt, welches eine isotonische Mineralsalzlösung, die dem Ionenmilieu und dem pH-Wert von Körperflüssigkeiten angepaßt ist und geeignete Nährstoffe enthält. Dieses Organbad wird mit einem Gasgemisch bestehend aus Sauerstoff und Kohlendioxyd begast, wobei der Kohlendioxydgehalt so bemessen ist, daß der pH-Wert des Organbades konstant bleibt. Die linken Vorhöfe werden in das Organbad eingespannt, die Spannung wird mittels eines Kraftaufnehmers registriert, wobei ein bestimmter Grundtonus eingestellt wird. Anschließend werden die linken Vorhöfe kontinuierlich in bestimmten Abständen elektrisch gereizt und die dabei erfolgenden Kontraktionen registriert. Nach Zugabe des Wirkstoffs werden die Kontraktionen weiterhin registriert. Eine Kontraktionsverstärkung um mindestens 25 % gilt als signifikante positiv-inotrope Wirkung.

Hervorgehoben seien solche Verbindungen der allgemeinen Formel (I), die bereits ab einer Konzentration von $10^{-5}$ g/ml am linken Vorhof des isolierten Meerschweinchenherzens in der folgenden Versuchsanordnung eine positiv inotrope Wirkung zeigen:

Beispielhaft seien erwähnt:

| | Δ dp/dt |
|---|---|
| Beispiel 2 | + 36 % |
| Beispiel 5 | + 25 % |
| Beispiel 7 | + 33 % |
| Beispiel 9 | + 40 % |

**Patentansprüche**

1. Dihydropyridin der allgemeinen Formel I

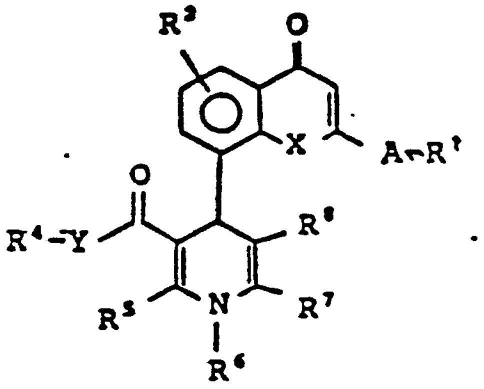

(I)

in welcher

$R^1$ für Wasserstoff, einen geradkettigen, cyclischen oder verzweigten aliphatischen Kohlenwasserstoff mit 1 bis 8 C-Atomen, einen Carbonsäurealkylester mit 1 bis 8 C-Atomen in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei die genannten Aryle bzw. Heteroaryle gegebenenfalls einen bis 5 gleiche oder verschiedene Substituenten aus der Gruppe Fluor, Chlor, Brom, Jod, Alkyl mit 1 bis 8 C-Atomen, Alkylthio mit 1 bis 8 C-Atomen, Alkylsulfinyl mit 1 bis 8 C-Atomen, Cyano, Hydroxy, Nitro, Mono- oder Polyfluoralkyl mit 1 bis 4 C-Atomen, Mono- oder Polyfluoralkoxy mit 1 bis 4 C-Atomen, Mono- oder Polyfluoralkylthio mit 1 bis 4 C-Atomen, Amino, Monoalkylamino mit 1 bis 5 C-Atomen, Dialkylamino mit jeweils 1 bis 5 C-Atomen aufweisen,

$R^2$ für Wasserstoff oder ein bis drei Fluor- oder Chloratome steht,

$R^4$ für einen geradkettigen, verzweigten oder cyclischen, gesättigten oder ungesättigten Kohlenwasserstoffrest mit 1 bis 18 C-Atomen steht, der gegebenenfalls substituiert ist durch Alkoxy mit 1 bis 8 C-Atomen, Alkylthio mit 1 bis 8 C-Atomen, Alkylsulfinyl mit 1 bis 8 C-Atomen, Trialkylsilyl mit jeweils 1 bis 5 C-Atomen, Cl, Br, J, F, Cyano, Hydroxy, Amino, Alkylamino mit 1 bis 5 C-Atomen, Dialkylamino mit jeweils 1 bis 5 C-Atomen, Morpholinyl, Piperidyl, Piperazinyl, Nitro, Nitrat, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl oder Chinazolyl, wobei die genannten Aromaten oder Heteroaromaten gegebenenfalls substituiert sein können durch 1 bis 3 gleiche oder verschiedene Substituenten aus der Gruppe F, Cl, Br, Alkyl mit 1 bis 5 C-Atomen, Alkoxy mit 1 bis 5 C-Atomen, Alkylthio mit 1 bis 4 C-Atomen, Alkylsulfinyl mit 1 bis 4 C-Atomen,

$R^5$ und $R^7$ gleich oder verschieden sein können und für Wasserstoff oder einen geradkettigen, verzweigten oder cyclischen, gesättigten oder ungesättigten aliphatischen Alkylrest mit 1 bis 8 C-Atomen stehen, der gegebenenfalls ein oder zwei gleiche oder verschiedene Heterokettenglieder aus der Gruppe O, CO, S oder N-Alkyl mit 1 bis 6 C-Atomen enthält und der gegebenenfalls substituiert ist durch Halogen, Nitro, Cyano, Hydroxy, Amino, Monoalkylamino oder Dialkylamino mit jeweils 1 bis 5 C-Atomen,

$R^6$ für Wasserstoff oder einen geradkettigen oder verzweigten Alkylrest mit 1 bis 16 C-Atomen steht, der gegebenenfalls substituiert ist durch Alkoxy mit 1 bis 8 C-Atomen, Halogen oder Morpholino,

$R^8$ für Wasserstoff, Nitro, Cyano, Fluor, Chlor, Brom, Alkyl mit 1 bis 3 C-Atomen, Mono- oder Polyfluoralkyl mit 1 bis 3 C-Atomen oder Hydroxycarbonyl steht,

A für eine einfache Bindung oder eine Alkylenkette mit 1 bis 18 C-Atomen steht, die gegebenenfalls durch O oder S unterbrochen sein kann,

X für O oder S steht und

Y für eine einfache Bindung, O, S, -NH- oder -N(Alkyl)- mit 1 bis 6 C-Atomen steht,

in Form von Isomeren, Isomerengemischen, Racematen und optischen Antipoden sowie ihre pharmazeutisch unbedenklichen Salze.

2. Verbindungen gemäß Anspruch 1, in denen

$R^1$ für Wasserstoff, einen geradkettigen, verzweigten oder cyclischen aliphatischen Kohlenwasserstoff mit 1 bis 7 C-Atomen, einen Carbonsäurealkylester mit 1 bis 6 C-Atomen in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl, Benzimidazolyl oder Chinazolyl steht, wobei die genannten Aryle bzw. Heteroaryle gegebenenfalls einen bis 4 gleiche oder verschiedene Substituenten aus der Gruppe Fluor, Chlor, Brom, Alkyl mit 1 bis 6 C-Atomen, Alkylthio mit 1 bis 6 C-Atomen, Alkylsulfinyl mit 1 bis 6 C-Atomen, Cyano, Hydroxy, Nitro, Mono- oder Polyfluoralkyl mit 1 bis 3 C-Atomen, Mono- oder Polyfluoralkoxy mit 1 bis 3 C-Atomen, Amino, Monoalkylamino mit 1 bis 4 C-Atomen, Dialkylamino mit jeweils 1 bis 4 C-Atomen aufweisen,

$R^2$ für Wasserstoff oder ein bis drei Fluoratome steht,

$R^4$ für einen geradkettigen, verzweigten oder cyclischen, gesättigten oder ungesättigten Kohlenwasserstoffrest mit 1 bis 12 C-Atomen steht, der gegebenenfalls substituiert ist durch Alkoxy mit 1 bis 6 C-Atomen, Alkylthio mit 1 bis 6 C-Atomen, Alkylsulfinyl mit 1 bis 6 C-Atomen, Trialkylsilyl mit jeweils 1 bis 3 C-Atomen, Cl, Br, F, Cyano, Hydroxy, Amino, Alkylamino mit 1 bis 4 C-Atomen, Dialkylamino mit jeweils 1 bis 4 C-Atomen, Morpholinyl, Piperidyl, Piperazinyl, Nitro, Nitrat, Phenyl, Naphthyl, Thienyl, Furyl, Pyrryl, Imidazolyl, Oxazolyl, Thiazolyl, Pyridyl, Pyrimidyl, Pyrazinyl, Indolyl, wobei die genannten Aromaten oder Heteroaromaten gegebenenfalls substituiert sein können durch 1 bis 3 gleiche oder verschiedene Substiuenten aus der Gruppe F, Cl, Br, Alkyl mit 1 bis 4 C-Atomen, Alkoxy mit 1 bis 4 C-Atomen, Alkylthio mit 1 bis 3 C-Atomen, Alkylsulfinyl mit 1 bis 3 C-Atomen,

$R^5$ und $R^7$ gleich oder verschieden sind und für Wasserstoff oder einen geradkettigen, verzweigten oder cyclischen, gesättigten oder ungesättigten aliphatischen Alkylrest mit 1 bis 6 C-Atomen stehen, der gegebenenfalls ein oder zwei gleiche oder verschiedene Heterokettenglieder aus der Gruppe O, CO, S oder N-Alkyl mit 1 bis 4 C-Atomen enthält und der gegebenenfalls substituiert ist durch Halogen, Nitro, Cyano, Hydroxy, Amino, Monoalkylamino mit 1 bis 4 C-Atomen oder Dialkylamino mit jeweils 1 bis 4 C-Atomen,

$R^6$ für Wasserstoff oder einen geradkettigen oder verzweigten Alkylrest mit 1 bis 12 C-Atomen steht, der gegebenenfalls substituiert ist durch Alkoxy mit 1 bis 6 C-Atomen, Halogen oder Morpholino,

$R^8$ für Nitro, Cyano, Fluor oder Chlor steht,

A für eine einfache Bindung oder eine Alkylenkette mit 1 bis 16 C-Atomen steht, die gegebenenfalls durch O oder S unterbrochen sein kann,

X für O oder S steht und

Y für eine einfache Bindung, O, S, -NH- oder -N(Alkyl)- mit 1 bis 4 C-Atomen steht.

3. Verbindungen gemäß Ansprüche 1 - 2 zur Bekämpfung von Erkrankungen.

4. Verbindungen gemäß Ansprüche 1 - 2 zur Verbesserung der Herzkontraktilität, als Antihypotonica, zur Senkung von Blutzucker, zur Abschwellung von Schleimhäuten, zur Beeinflussung des Salz- und/oder Flüssigkeitshaushaltes.

5. Verfahren zur Herstellung von Dihydropyridinen der allgemeinen Formel (I)

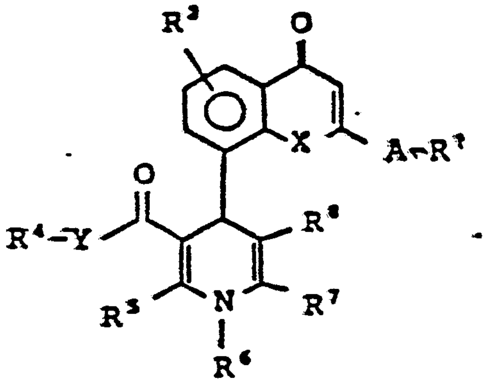

(I)

in welcher

$R^1$ für Wasserstoff, einen geradkettigen, cyclischen oder verzweigten aliphatischen Kohlenwasserstoff mit 1 bis 8 C-Atomen, einen Carbonsäurealkylester mit 1 bis 8 C-Atomen in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei die genannten Aryle bzw. Heteroaryle gegebenenfalls einen bis 5 gleiche oder verschiedene Substituenten aus der Gruppe Fluor, Chlor, Brom, Jod, Alkyl mit 1 bis 8 C-Atomen, Alkylthio mit 1 bis 8 C-Atomen, Alkylsulfinyl mit 1 bis 8 C-Atomen, Cyano, Hydroxy, Nitro, Mono- oder Polyfluoralkyl mit 1 bis 4 C-Atomen, Mono- oder Polyfluoralkoxy mit 1 bis 4 C-Atomen, Mono- oder Polyfluoralkylthio mit 1 bis 4 C-Atomen, Amino, Monoalkylamino mit 1 bis 5 C-Atomen, Dialkylamino mit jeweils 1 bis 5 C-Atomen aufweisen,

$R^2$ für Wasserstoff oder ein bis drei Fluor- oder Chloratome steht,

$R^4$ für einen geradkettigen, verzweigten oder cyclischen, gesättigten oder ungesättigten Kohlenwasserstoffrest mit 1 bis 18 C-Atomen steht, der gegebenenfalls substituiert ist durch Alkoxy mit 1 bis 8 C-Atomen, Alkylthio mit 1 bis 8 C-Atomen, Alkylsulfinyl mit 1 bis 8 C-Atomen, Trialkylsilyl mit jeweils 1 bis 5 C-Atomen, Cl, Br, J, F, Cyano, Hydroxy, Amino, Alkylamino mit 1 bis 5 C-Atomen, Dialkylamino mit jeweils 1 bis 5 C-Atomen, Morpholinyl, Piperidyl, Piperazinyl, Nitro, Nitrat, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl oder Chinazolyl, wobei die genannten Aromaten oder Heteroaromaten gegebenenfalls substituiert sein können durch 1 bis 3 gleiche oder verschiedene Substituenten aus der Gruppe F, Cl, Br, Alkyl mit 1 bis 5 C-Atomen, Alkoxy mit 1 bis 5 C-Atomen, Alkylthio mit 1 bis 4 C-Atomen, Alkylsulfinyl mit 1 bis 4 C-Atomen,

$R^5$ und $R^7$ gleich oder verschieden sein können und für Wasserstoff oder einen geradkettigen, verzweigten oder cyclischen, gesättigten oder ungesättigten aliphatischen Alkylrest mit 1 bis 8 C-Atomen stehen, der gegebenenfalls ein oder zwei gleiche oder verschiedene Heterokettenglieder aus der Gruppe O, CO, S oder N-Alkyl mit 1 bis 6 C-Atomen enthält und der gegebenenfalls substituiert ist durch Halogen, Nitro, Cyano, Hydroxy, Amino, Monoalkylamino oder Dialkylamino mit jeweils 1 bis 5 C-Atomen,

$R^6$ für Wasserstoff oder einen geradkettigen oder verzweigten Alkylrest mit 1 bis 16 C-Atomen steht, der gegebenenfalls substituiert ist durch Alkoxy mit 1 bis 8 C-Atomen, Halogen oder Morpholino,

$R^8$ für Wasserstoff, Nitro, Cyano, Fluor, Chlor, Brom, Alkyl mit 1 bis 3 C-Atomen, Mono- oder Polyfluoralkyl mit 1 bis 3 C-Atomen oder Hydroxycarbonyl steht,

A für eine einfache Bindung oder eine Alkylenkette mit 1 bis 18 C-Atomen steht, die gegebenenfalls durch O oder S unterbrochen sein kann,

X für O oder S steht und

Y für eine einfache Bindung, O, S, -NH- oder -N(Alkyl)- mit 1 bis 6 C-Atomen steht,

dadurch gekennzeichnet, daß man

A) Aldehyde der Formel II

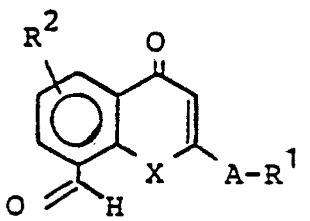

(II)

in welcher $R^1$, $R^2$, A und X die oben angegebene Bedeutung haben mit Enaminen der allgemeinen Formel III

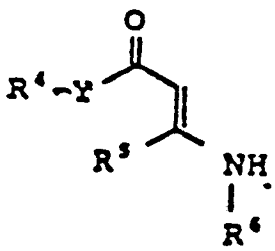

(III)

in welcher $R^4$, $R^5$, $R^6$ und Y die oben angegebene Bedeutung haben und Ketonen der allgemeinen Formel IV

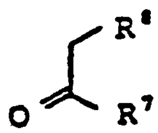

(IV)

in welcher $R^7$ und $R^8$ die oben angegebene Bedeutung haben, gegebenenfalls in Gegenwart inerter organischer Lösungsmittel bei Temperaturen zwischen 20 und 150°C umsetzt, oder

B) Aldehyde der allgemeinen Formel II in welcher $R^1$, $R^2$, A und X die oben angegebene Bedeutung haben mit Ketonen der allgemeinen Formel V

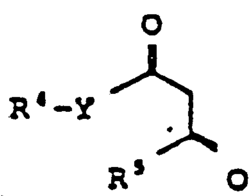

(V)

in welcher $R^4$, $R^5$ und Y die oben angegebene Bedeutung haben und Enaminen der allgemeinen Formel VI

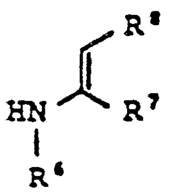

VI

(VI)

in welcher $R^6$, $R^7$ und $R^8$ die oben angegebene Bedeutung haben, gegebenenfalls in Gegenwart inerter organischer Lösungsmittel bei Temperaturen zwischen 20 und 150°C umsetzt, oder

C) Ylidenverbindungen der allgemeinen Formel VII

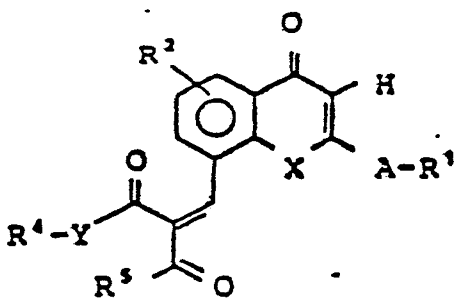

(VII)

in welcher $R^1$, $R^2$, $R^4$, $R^5$, A, X und Y die oben angegebene Bedeutung haben, mit Enaminen der allgemeinen Formel VI, in welcher $R^6$, $R^7$ und $R^8$ die oben angegebene Bedeutung haben, gegebenenfalls in Gegenwart inerter organischer Lösungsmittel bei Temperaturen zwischen 20 und 150° C umsetzt, oder

D) Benzylidenverbindungen der allgemeinen Formel VIII

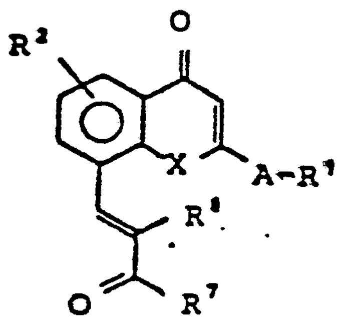

(VIII)

in welcher $R^1$, $R^2$, $R^7$, $R^8$, A und X die oben angegebene Bedeutung haben mit Enaminen der allgemeinen Formel III in welcher $R^4$, $R^5$, $R^6$ und Y die oben angegebene Bedeutung haben, gegebenenfalls in inerten organischen Lösungsmitteln bei Temperaturen von 20 bis 150° C umsetzt.

6. Arzneimittel enthaltend mindestens eine Verbindungen der Formel I gemäß Ansprüchen 1 - 2.

7. Verfahren zur Herstellung von Arzneimitteln, dadurch gekennzeichnet, daß man Verbindungen der Formel I gemäß Ansprüchen 1 - 2 gegebenenfalls unter Verwendung von üblichen Hilfs- und Trägerstoffen in eine geeignete Applikationsform überführt.

8. Verwendung der Verbindungen gemäß Ansprüchen 1 - 2 bei der Herstellung von Arzneimitteln zur Verbesserung der Herzkontraktilität, zur Erhöhung des Blutdruckes, zur Senkung von Blutzucker, zur Abschwellung von Schleimhäuten, zur Beeinflussung des Salz- und/oder Flüssigkeitshaushaltes.

**Claims**

1. Dihydropyridine of the general formula I

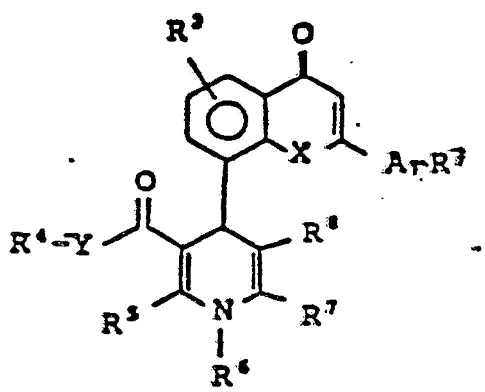

(I)

in which

$R^1$ represents hydrogen, a straight-chain, cyclic or branched aliphatic hydrocarbon with 1 to 8 C atoms, a carboxylic acid alkyl ester with 1 to 8 C atoms in the alkyl chain, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl or quinoxalyl, where the aryl and heteroaryl radicals mentioned optionally contain one to 5 identical or different substituents from the group comprising fluorine, chlorine, bromine, iodine, alkyl with 1 to 8 C atoms, alkylthio with 1 to 8 C atoms, alkylsulphinyl with 1 to 8 C atoms, cyano, hydroxyl, nitro, mono- or poly-fluoroalkyl with 1 to 4 C atoms, mono- or poly-fluoroalkoxy with 1 to 4 C atoms, mono- or poly-fluoroalkylthio with 1 to 4 C atoms, amino, monoalkylamino with 1 to 5 C atoms and dialkylamino with in each case 1 to 5 C atoms,

$R^2$ represents hydrogen or one to three fluorine or chlorine atoms,

$R^4$ represents a straight-chain, branched or cyclic saturated or unsaturated hydrocarbon radical with 1 to 18 C atoms, which is optionally substituted by alkoxy with 1 to 8 C atoms, alkylthio with 1 to 8 C atoms, alkylsulphinyl with 1 to 8 C atoms, trialkylsilyl with in each case 1 to 5 C atoms, Cl, Br, I, F, cyano, hydroxyl, amino, alkylamino with 1 to 5 C atoms, dialkylamino with in each case 1 to 5 C atoms, morpholinyl, piperidyl, piperazinyl, nitro, nitrate, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl or quinazolyl, it being possible for the aromatics or heteroaromatics mentioned optionally to be substituted by 1 to 3 identical or different substituents from the group comprising F, Cl, Br, alkyl with 1 to 5 C atoms, alkoxy with 1 to 5 C atoms, alkylthio with 1 to 4 C atoms and alkylsulphinyl with 1 to 4 C atoms,

$R^5$ and $R^7$ can be identical or different and represent hydrogen or a straight-chain, branched or cyclic, saturated or unsaturated aliphatic alkyl radical with 1 to 8 C atoms, which optionally contains one or two identical or different hetero chain members from the group comprising O, CO, S or N-alkyl with 1 to 6 C atoms, and is optionally substituted by halogen, nitro, cyano, hydroxyl, amino or monoalkylamino or dialkylamino with in each case 1 to 5 C atoms,

$R^6$ represents hydrogen or a straight-chain or branched alkyl radical with 1 to 16 C atoms, which is optionally substituted by alkoxy with 1 to 8 C atoms halogen or morpholino,

$R^8$ represents hydrogen nitro cyano, fluorine, chlorine, bromine, alkyl with 1 to 3 C atoms, mono- or poly-fluoroalkyl with 1 to 3 C atoms or hydroxycarbonyl,

A represents a single bond or an alkylene chain with 1 to 18 C atoms, which can optionally be interrupted by O or S,

X represents O or S and

Y represents a single bond, O, S, -NH- or -N(alkyl)- with 1 to 6 C atoms,

in the form of isomers, isomer mixtures, racemates and optical antipodes, and their pharmaceutically acceptable salts.

2. Compounds according to Claim 1,

in which

$R^1$ represents hydrogen, a straight-chain, branched or cyclic aliphatic hydrocarbon with 1 to 7 C atoms, a carboxylic acid alkyl ester with 1 to 6 C atoms in the alkyl chain, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl, benzimidazolyl or quinazolyl, where the aryl and heteroaryl radicals mentioned optionally contain one to 4 identical or different substituents from the group comprising fluorine, chlorine, bromine, alkyl with 1 to 6 C atoms, alkylthio with 1 to 6 C atoms, alkylsulphinyl with 1 to 6 C atoms, cyano, hydroxyl, nitro, mono- or poly-fluoroalkyl with 1 to 3 C atoms, mono- or poly-fluoroalkoxy with 1 to 3 C atoms, amino, monoalkylamino with 1 to 4 C atoms and dialkylamino with in each case 1 to 4 C atoms,

$R^2$ represents hydrogen or one to three fluorine atoms,

$R^4$ represents a straight-chain, branched or cyclic, saturated or unsaturated hydrocarbon radical with 1 to 12 C atoms, which is optionally substituted by alkoxy with 1 to 6 C atoms, alkylthio with 1 to 6 C atoms, alkylsulphinyl with 1 to 6 C atoms, trialkylsilyl with in each case 1 to 3 C atoms, Cl, Br, F, cyano, hydroxyl, amino, alkylamino with 1 to 4 C atoms, dialkylamino with in each case 1 to 4 C atoms, morpholinyl, piperidyl, piperazinyl, nitro, nitrate, phenyl, naphthyl, thienyl, furyl, pyrryl, imidazolyl, oxazolyl, thiazolyl, pyridyl, pyrimidyl, pyrazinyl or indolyl, it being possible for the aromatics or heteroaromatics mentioned optionally to be substituted by 1 to 3 identical or different substituents from the group comprising F, Cl, Br, alkyl with 1 to 4 C atoms, alkoxy with 1 to 4 C atoms, alkylthio with 1 to 3 C atoms and alkylsulphinyl with 1 to 3 C atoms,

$R^5$ and $R^7$ are identical or different and represent hydrogen or a straight-chain, branched or cyclic, saturated or unsaturated aliphatic alkyl radical with 1 to 6 C atoms, which optionally contains one or two identical or different hetero chain members from the group comprising O, CO, S and N-alkyl with 1 to 4 C atoms, and is optionally substituted by halogen, nitro, cyano, hydroxyl, amino, monoalkylamino with 1 to 4 C atoms or dialkylamino with in each case 1 to 4 C atoms,

$R^6$ represents hydrogen or a straight-chain or branched alkyl radical with 1 to 12 C atoms, which is optionally substituted by alkoxy with 1 to 6 C atoms, halogen or morpholino,

$R^8$ represents nitro, cyano, fluorine or chlorine,

A represents a single bond or an alkylene chain with 1 to 16 C atoms, which can optionally be interrupted by O or S,

X represents O or S and

Y represents a single bond, O, S, -NH- or -N(alkyl)- with 1 to 4 C atoms.

3. Compounds according to Claims 1 - 2 for combating diseases.

4. Compounds according to Claims 1 - 2 for improving heart contractility, as antihypotonic agents, for lowering the blood sugar level, for detumescing mucous membranes and for influencing the salt and/or liquid balance.

5. Process for the preparation of dihydropyridines of the general formula I

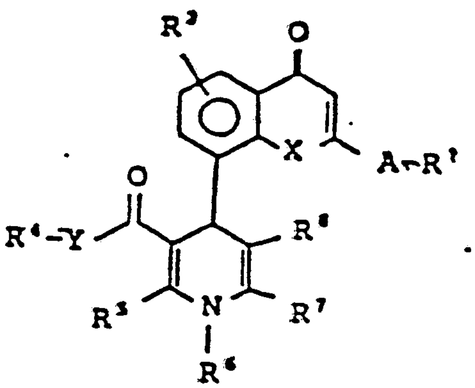

(I)

in which

$R^1$ represents hydrogen, a straight-chain, cyclic or branched aliphatic hydrocarbon with 1 to 8 C atoms, a carboxylic acid alkyl ester with 1 to 8 C atoms in the alkyl chain, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl or quinoxalyl, where the aryl and heteroaryl radicals mentioned optionally contain one to 5 identical or different substituents from the group comprising fluorine, chlorine, bromine, iodine, alkyl with 1 to 8 C atoms, alkylthio with 1 to 8 C atoms, alkylsulphinyl with 1 to 8 C atoms, cyano, hydroxyl, nitro, mono- or poly-fluoroalkyl with 1 to 4 C atoms, mono- or poly-fluoroalkoxy with 1 to 4 C atoms, mono- or poly-fluoroalkylthio with 1 to 4 C atoms, amino, monoalkylamino with 1 to 5 C atoms and dialkylamino with in each case 1 to 5 C atoms,

$R^2$ represents hydrogen or one to three fluorine or chlorine atoms,

$R^4$ represents a straight-chain, branched or cyclic saturated or unsaturated hydrocarbon radical with 1 to 18 C atoms, which is optionally substituted by alkoxy with 1 to 8 C atoms, alkylthio with 1 to 8 C atoms, alkylsulphinyl with 1 to 8 C atoms, trialkylsilyl with in each case 1 to 5 C atoms, Cl, Br, I, F, cyano, hydroxyl, amino, alkylamino with 1 to 5 C atoms, dialkylamino with in each case 1 to 5 C atoms, morpholinyl, piperidyl, piperazinyl, nitro, nitrate, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl or quinazolyl, it being possible for the aromatics or heteroaromatics mentioned optionally to be substituted by 1 to 3 identical or different substituents from the group comprising F, Cl, Br, alkyl with 1 to 5 C atoms, alkoxy with 1 to 5 C atoms, alkylthio with 1 to 4 C atoms and alkylsulphinyl with 1 to 4 C atoms,

$R^5$ and $R^7$ can be identical or different and represent hydrogen or a straight-chain, branched or cyclic, saturated or unsaturated aliphatic alkyl radical with 1 to 8 C atoms, which optionally contains one or two identical or different hetero chain members from the group comprising O, CO, S or N-alkyl with 1 to 6 C atoms, and is optionally substituted by halogen, nitro, cyano, hydroxyl, amino or monoalkylamino or dialkylamino with in each case 1 to 5 C atoms,

$R^6$ represents hydrogen or a straight-chain or branched alkyl radical with 1 to 16 C atoms, which is optionally substituted by alkoxy with 1 to 8 C atoms, halogen or morpholino,

$R^8$ represents hydrogen, nitro, cyano, fluorine, chlorine, bromine, alkyl with 1 to 3 C atoms, mono- or poly-fluoroalkyl with 1 to 3 C atoms or hydroxycarbonyl,

A represents a single bond or an alkylene chain with 1 to 18 C atoms, which can optionally be interrupted by O or S,

X represents O or S and

Y represents a single bond, O, S, -NH- or -N(alkyl)- with 1 to 6 C atoms

characterised in that

A) aldehydes of the formula II

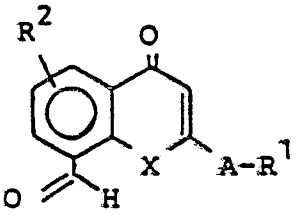

(II)

in which
$R^1$, $R^2$, A and X have the abovementioned meaning are reacted with enamines of the general formula III

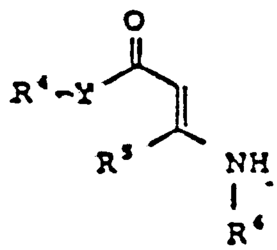

(III)

in which
$R^4$, $R^5$, $R^6$ and Y have the abovementioned meaning and ketones of the general formula IV

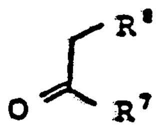

(IV)

in which
$R^7$ and $R^8$ have the abovementioned meaning, if appropriate in the presence of inert organic solvents at temperatures between 20 and 150°C, or

B) aldehydes of the general formula II in which $R^1$, $R^2$, A and X have the abovementioned meaning are reacted with ketones of the general formula V

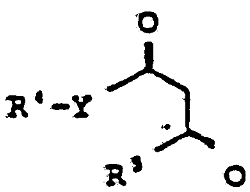

(V)

in which
$R^4$ and $R^5$ and Y have the abovementioned meaning, and enamines of the general formula VI

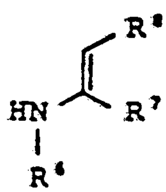

VI (VI)

in which
$R^6$, $R^7$ and $R^8$ have the abovementioned meaning, if appropriate in the presence of inert organic solvents at temperatures between 20 and 150°C or

C) ylidene compounds of the general formula VII

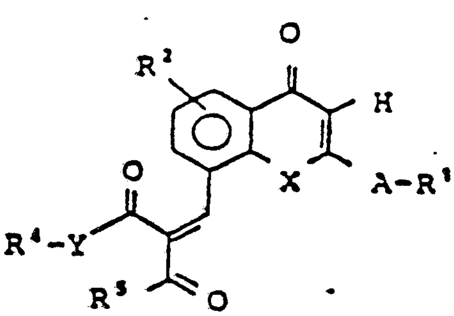

(VII)

in which
$R^1$, $R^2$, $R^4$, $R^5$, A, X and Y have the abovementioned meaning,
are reacted with enamines of the general formula VI in which
$R^6$, $R^7$ and $R^8$ have the abovementioned meaning, if appropriate in the presence of inert organic solvents at temperatures between 20 and 150°C or
D) benzylidene compounds of the general formula VIII

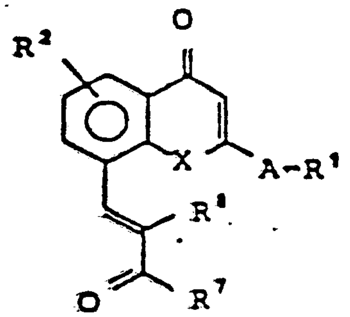

(VIII)

in which
$R^1$, $R^2$, $R^7$, $R^8$, A and X have the abovementioned meaning,
are reacted with enamines of the general formula III in which
$R^4$, $R^5$, $R^6$ and Y have the abovementioned meaning, if appropriate in inert organic solvents at temperatures between 20 and 150°C.

6. Medicaments containing at least one compound of the formula I according to Claims 1 - 2.

7. Process for the preparation of medicaments, characterised in that compounds of the formula I according to Claims 1 - 2 are converted into a suitable administration form, if appropriate using the customary auxiliaries and excipients.

8. Use of the compounds according to Claims 1 - 2 for preparing medicaments for improving heart contractility, for increasing the blood pressure, for lowering the blood sugar level, for detumescing mucous membranes and for influencing the salt and/or liquid balance.

**Revendications**

1. Dihydropyridines répondant à la formule générale (I)

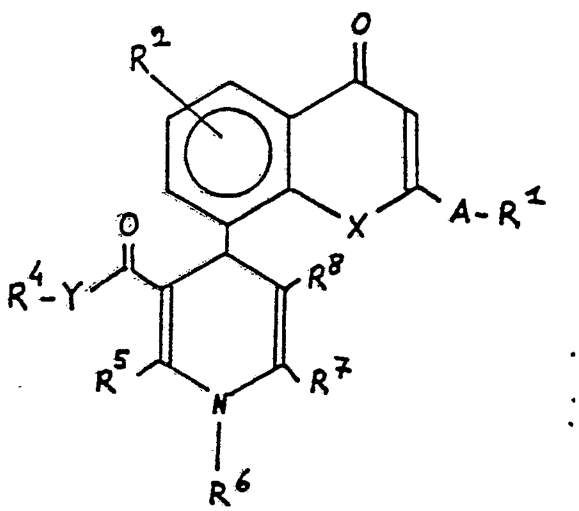

(I)

dans laquelle
$R^1$ représente de l'hydrogène, un hydrocarbure aliphatique à chaîne droite ou ramifiée ou cyclique à 1 à 8 atomes de carbone, un ester alkylique d'un acide carboxylique contenant de 1 à 8 atomes de carbone dans la chaîne alkylique, un radical phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, isoquinolyle, indolyle, benzimidazolyle, quinazolyle ou quinoxalyle où les radicaux aryle ou hétéroalyle précités sont éventuellement substitués par 1 à 5 substituants identiques ou différents, choisis dans le groupe fluor, chlore, brome, iode,

alkyle (1 à 8 atomes de carbone), alkylthio (1 à 8 atomes de carbone), alkylsulfinyle (1 à 8 atomes de carbone), cyano, hydroxy, nitro, mono- ou polyfluoralkyle (1 à 4 atomes de carbone), mono- ou polyfluoralcoxy (1 à 4 atomes de carbone), mono- ou polyfluoralkylthio (1 à 4 atomes de carbone), amino, monoalkylamino (1 à 5 atomes de carbone) et dialkylamino (contenant chacun 1 à 5 atomes de carbone),

$R^2$ représente de l'hydrogène ou un à trois atomes de fluor ou de chlore,

$R^4$ représente un radical d'hydrocarbure saturé ou insaturé à chaîne droite ou ramifiée ou cyclique à 1 à 18 atomes de carbone, qui est éventuellement substitué par un radical alcoxy (à 1 à 8 atomes de carbone), alkylthio (à 1 à 8 atomes de carbone), alkylsulfinyle (à 1 à 8 atomes de carbone), trialkylsilyle (avec chaque fois 1 à 5 atomes de carbone), Cl, Br, I, F, cyano- hydroxy, amino, alkylamino (à 1 à 5 atomes de carbone), dialkylamino (avec chaque fois 1 à 5 atomes de carbone), morpholinyle, piperidyle, piperazinyle, nitro, nitrate, phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, indolyle ou quinazolyle, tandis que les hydrocarbures aromatiques ou hétéro-aromatiques précités peuvent être éventuellement substitués par un à trois substituants identiques ou différents, choisis dans le groupe F, Cl, Br, alkyle (à 1 à 5 atomes de carbone), alcoxy (à 1 à 5 atomes de carbone), alkylthio (à 1 à 4 atomes de carbone) alkylsulfinyle (à 1 à 4 atomes de carbone),

$R^5$ et $R^7$ peuvent être identiques ou différents et représentent de l'hydrogène ou un radical alkyle aliphatique saturé ou insaturé à chaîne droite ou ramifiée ou cyclique (à 1 à 8 atomes de carbone), qui contient éventuellement un ou deux hétéro-éléments de chaîne identiques ou différents, choisis dans le groupe O, CO, S ou N-alkyle (1 à 6 atomes de carbone) et qui est éventuellement substitué par un halogène, un radical nitro, cyano, hydroxy, amino, monoalkylamino ou dialkylamino (avec chaque fois 1 à 5 atomes de carbone),

$R^6$ représente de l'hydrogène ou un radical alkyle à chaîne droite ou ramifiée (à 1 à 16 atomes de carbone), qui est éventuellement substitué par un radical alcoxy (à 1 à 8 atomes de carbone), un halogène ou un groupe morpholino,

$R^8$ représente de l'hydrogène, un groupe nitro ou cyano, du fluor, du chlore, du brome, un alkyle (à 1 à 3 atomes de carbone), un mono- ou polyfluoroalkyle (1 à 3 atomes de carbone) ou hydroxycarbonyle,

A représente une liaison simple ou une chaîne alkylénique (à 1 à 18 atomes de carbone) qui peut éventuellement être interrompue par O ou S,

X représente O ou S, et

Y représente une liaison simple, O, S, -NH- ou -N(alkyle) (à 1 à 6 atomes de carbone),

sous forme d'isomères, de mélanges d'isomères, de racémates et d'antipodes optiques, ainsi que de leurs sels utilisables en pharmacie.

2. Composés selon la revendication 1, dans lesquels

$R^1$ représente de l'hydrogène, un radical d'hydrocarbure aliphatique à chaîne droite ou ramifiée ou cyclique, à 1 à 7 atomes de carbone, un ester alkylique d'un acide carboxylique (à 1 à 6 atomes de carbone) dans le radical alkyle, un radical phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, indolyle, benzimidazolyle ou quinazolyle, tandis que les aryles ou hétéroaryles précités sont éventuellement substitués par 1 à 4 substituants identiques ou différents, choisis dans le groupe fluor, chlore, brome, alkyle (1 à 6 atomes de carbone), alkylthio (1 à 6 atomes de carbone) alkylsulfinyle (1 à 6 atomes de carbone), cyano, hydroxy, nitro, mono- ou polyfluoralkyle (1 à 3 atomes de carbone), mono- ou polyfluoralcoxy (1 à 4 atomes de carbone), dialkylamino (avec chaque fois de 1 à 4 atomes de carbone),

$R^2$ représente de l'hydrogène ou un à trois atomes de fluor,

$R^4$ représente un radical d'hydrocarbure saturé ou insaturé à chaîne droite, ramifiée ou cyclique, avec 1 à 12 atomes de carbone, qui est éventuellement substitué par un radical alcoxy (à 1 à 6 atomes de carbone), alkylthio (à 1 à 6 atomes de carbone), alkylsulfinyle (1 à 6 atomes de carbone), trialkylsilyle (avec chaque fois 1 à 3 atomes de carbone), Cl, Br, F, Cyano, hydroxy, amino, alkylamino (à 1 à 4 atomes de carbone), dialkylamino (avec chaque fois 1 à 4 atomes de carbone), morpholinyle, piperidyle, piperazinyle, nitro, nitrate, phényle, naphtyle, thiényle, furyle, pyrryle, imidazolyle, oxazolyle, thiazolyle, pyridyle, pyrimidyle, pyrazinyle, indolyle, les hydrocarbures aromatiques ou hétéro-aromatiques précités pouvant éventuellement être substitués par un à trois substituants identiques ou différents, choisis dans le groupe F, Cl, Br, alkyle (à 1 à 4 atomes de carbone), alcoxy (à 1 à 4 atomes de carbone), alkylthio (à 1 à 3 atomes de carbone), alkylsulfinyle (à 1 à 3 atomes de carbone),

$R^5$ et $R^7$ sont identiques ou différents et représentent de l'hydrogène ou un radical alkylique saturé ou insaturé, à chaîne droite, ramifiée ou cyclique (à 1 à 6 atomes de carbone), qui contient éventuellement un ou deux hétéro-éléments le chaîne identiques ou différents, choisis dans le groupe O, CO, S ou N-alkyle (1 à 4 atomes de carbone), qui est éventuellement substitué par un halogène, nitro, cyano, hydroxy, amino, monoalkylamino (à 1 à 4 atomes de carbone) et dialkylamino (avec chaque fois 1 à 4 atomes de carbone),

$R^6$ représente de l'hydrogène ou un radical alkyle à chaîne droite ou ramifiée (à 1 à 12 atomes de carbone), qui est éventuellement substitué par un alcoxy (à 1 à 6 atomes de carbone), un halogène ou un morpholino,

$R^8$ représente un nitro, cyano, fluor ou chlore,

A représente une liaison simple ou une chaîne alkylénique (1 à 16 atomes de carbone) qui peut éventuellement être interrompue par O ou S,

X représente O ou S

Y représente une liaison simple, O, S, -NH- ou -N(alkyle) (à 1 à 4 atomes de carbone).

3. Composés selon les revendications 1 - 2, pour lutter contre la maladie.

4. Composés selon les revendications 1 - 2, pour améliorer la contractilité du coeur, comme anti-hypotonique, pour diminuer le sucre sanguin, pour décongestionner les muqueuses, pour influencer le métabolisme des sels et/ou des liquides.

5. Procédé de préparation des dihydropyridines répondant à la formule générale (I)

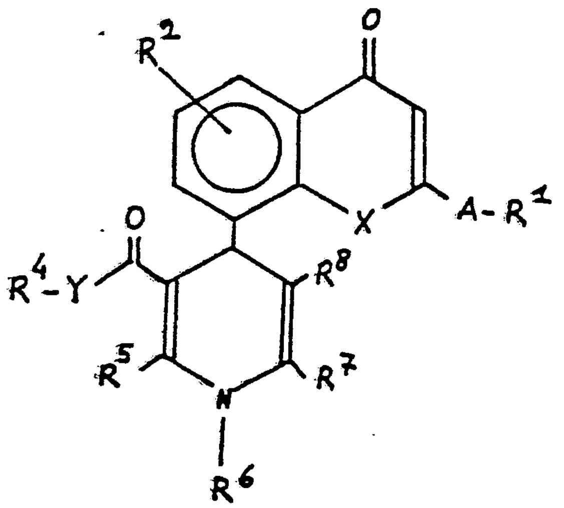

(I)

dans laquelle

$R^1$ représente de l'hydrogène, un hydrocarbure aliphatique à chaîne droite ou ramifiée ou cyclique à 1 à 8 atomes de carbone, un ester alkylique d'un acide carboxylique contenant de 1 à 3 atomes de carbone dans la chaîne alkylique, un radical phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, isoquinolyle, indolyle, benzimidazolyle, quinazolyle ou quinoxalyle où les radicaux aryle ou hétéroalyle précités sont éventuellement substitués par 1 à 5 substituants identiques ou différents, choisis dans le groupe fluor, chlore, brome, iode, alkyle (1 à 8 atomes de carbone), alkylthio (1 à 8 atomes de carbone), alkylsulfinyle (1 à 8 atomes de carbone), cyano, hydroxy, nitro, mono- ou polyfluoralkyle (1 à 4 atomes de carbone), mono- ou polyfluoralcoxy (1 à 4 atomes de carbone), mono- ou polyfluoralkylthio (1 à 4 atomes de carbone), amino, monoalkylamino (1 à 5 atomes de carbone) et dialkylamino (contenant chacun 1 à 5 atomes de carbone),

$R^2$ représente de l'hydrogène ou un à trois atomes de fluor ou de chlore,

$R^4$ représente un radical d'hydrocarbure saturé ou insaturé à chaîne droite ou ramifiée ou cyclique à 1 à 18 atomes de carbone, qui est éventuellement substitué par un radical alcoxy (à 1 à 8 atomes de carbone), alkylthio (à 1 à 8 atomes de carbone), alkylsulfinyle (à 1 à 8 atomes de carbone), trialkylsilyle (avec chaque fois 1 à 5 atomes de carbone), Cl, Br, I, F, cyano- hydroxy, amino, alkylamino (à 1 à 5 atomes de carbone), dialkylamino (avec chaque fois 1 à 5 atomes de carbone), morpholinyle, piperidyle, piperazinyle, nitro, nitrate, phényle, naphtyle, thiényle, furyle, pyrryle, pyrazolye, imidazolyle, oxazolyle, isoxazolyle, thiazolyle, pyridyle, pyridazinyle, pyrimidyle, pyrazinyle, quinolyle, indolyle ou quinazolyle, tandis que les hydrocarbures aromatiques ou hétéro-aromatiques précités peuvent être éventuellement substitués par un à trois substituants identiques ou différents, choisis dans le groupe F, Cl, Br, alkyle (à 1 à 5 atomes de carbone), alcoxy (à 1 à 5 atomes de carbone), alkylthio (à 1 à 4 atomes de carbone), alkylsulfinyl (à 1 à 4 atomes de carbone),

$R^5$ et $R^7$ peuvent être identiques ou différents et représentent de l'hydrogène ou un radical alkyle aliphatique saturé ou insaturé à chaîne droite ou ramifiée ou cyclique (à 1 à 8 atomes de carbone), qui contient éventuellement un ou deux hétéro-éléments de chaîne identiques ou différents, choisis dans le groupe O, CO, S ou N-alkyle (1 à 6 atomes de carbone) et qui est éventuellement substitué par un halogène, un radical nitro, cyano, hydroxy, amino, monoalkylamino ou dialkylamino (avec chaque fois 1 à 5 atomes de carbone),

$R^6$ représente de l'hydrogène ou un radical alkyle à chaîne droite ou ramfiée (à 1 à 16 atomes de carbone), qui est éventuellement substitué par un radical alcoxy (à 1 à 8 atomes de carbone), un halogène ou un groupe morpholino,

$R^8$ représente de l'hydrogène, un groupe nitro ou cyano, du fluor, du chlore, du brome, un alkyle (à 1 à 3 atomes de carbone), un mono- ou polyfluoroalkyle (1 à 3 atomes de carbone), ou hydroxycarbonyle,

A représente une liaison simple ou une chaîne alkylénique (à 1 à 18 atomes de carbone) qui peut éventuellement être interrompue par O ou S,

X représente O ou S, et

Y représente une liaison simple, O, S, -NH- ou -N(alkyle) - (à 1 à 6 atomes de carbone),

caractérisé en ce que

A) des aldéhydes répondant à la formule générale (II)

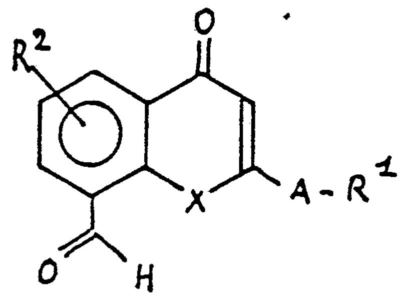

(II)

dans laquelle $R^1$, $R^2$, A et X ont les significations mentionnées ci-dessus sont mis en réaction avec des énamines répondant à la formule générale (III)

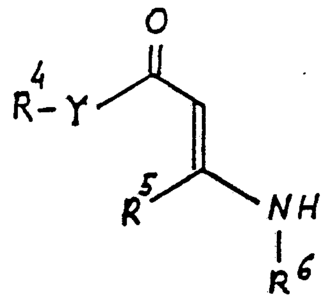

(III)

dans laquelle $R^4$, $R^5$, $R^6$ et Y ont les significations mentionnées ci-dessus et avec des cétones répondant à la formule générale (IV), dans laquelle

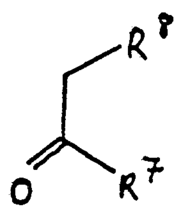

(IV)

$R^7$ et $R^8$ ont les significations mentionnées ci-dessus éventuellement en présence de solvant organiques inertes à des températures comprises entre 20 et 150° C, ou

B) des aldéhydes répondant à la formule générale (II) dans laquelle $R^1$, $R^2$, A et X ont les significations mentionnées ci-dessus, sont mis en réaction avec des cétones répondant à la formule génélrale (V)

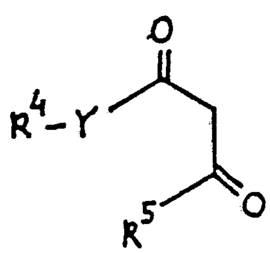

(V)

dans laquelle $R^4$, $R^5$ et Y ont les significations mentionnées ci-dessus et avec des énamines répondant à la formule générale (VI)

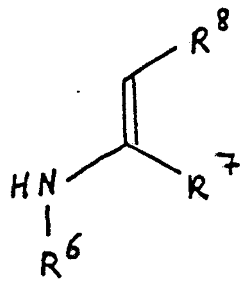

(VI)

dans laquelle $R^6$, $R^7$ et $R^8$ ont la signification mentionnée ci-dessus, éventuellement en présence de solvants organiques inertes à des températures comprises entre 20 et 150° C, ou

C) des composés ylidènes répondant à la formule générale (VII)

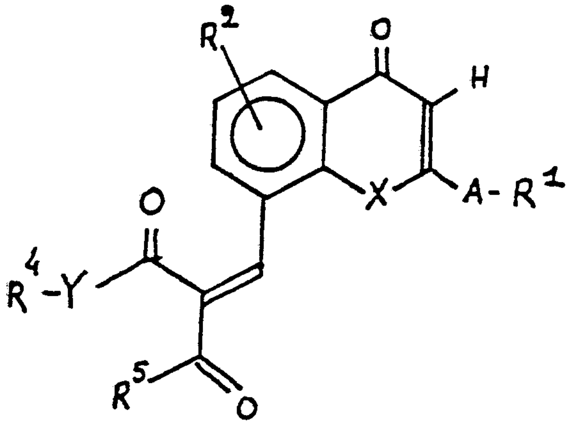

(VII)

dans laquelle $R^1$, $R^2$, $R^4$, $R^5$, A, X, et Y ont les significations mentionnées ci-dessus, sont mis en réaction avec des énamines répondant à la formule générale (VI) dans laquelle $R^6$, $R^7$ et $R^8$ ont les significations mentionnées ci-dessus, éventuellement en présence de solvants organiques inertes à des températures comprises entre 20 et 150° C

ou

des composés benzylidènes répondant à la formule générale (VIII)

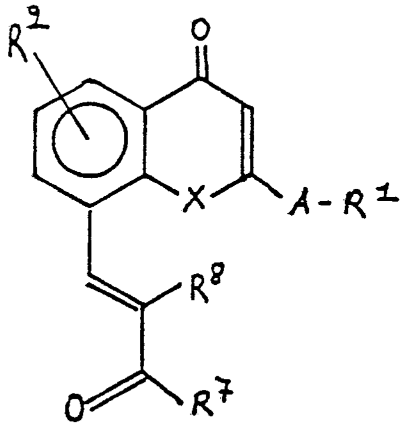

(VIII)

dans laquelle $R^1$, $R^2$, $R^7$, $R^8$, A et X ont les significations mentionnées ci-dessus, sont mis en réaction avec des énamines répondant à la formule générale (III), dans laquelle $R^4$, $R^5$, $R^6$ et Y ont les significations mentionnées ci-dessus, éventuellement en présence de solvants organiques inertes à des températures comprises entre 20 et 150° C.

6. Médicaments contenant au moins un composé de la formule (I) et selon les revendications 1 - 2.

7. Procédé de production de médicaments, caractérisé en ce que l'on transforme des composés de la formule (I) selon les revendications 1 - 2, éventuellement en utilisant des excipients ou des adjuvants usuels sous une forme appropriée pour l'administration.

8. Utilisation des composés selon les revendications 1 - 2 pour la production de médicaments pour améliorer la contractilité du coeur, pour augmenter la pression sanguine, pour diminuer le sucre sanguin, pour décongestionner les muqueuses, pour influencer le métabolisme des sels et/ou des liquides.